(19) Europäisches Patentamt
European Patent Office
Office européen des brevets

(11) **EP 4 679 455 A1**

(12) **EUROPEAN PATENT APPLICATION**
published in accordance with Art. 153(4) EPC

(43) Date of publication:
**14.01.2026 Bulletin 2026/03**

(21) Application number: 24728854.1

(22) Date of filing: **05.02.2024**

(51) International Patent Classification (IPC):
*G21C 17/112* (2006.01)   *G21C 17/108* (2006.01)
*G01K 7/02* (2021.01)   *G01K 17/10* (2006.01)
*G01T 3/00* (2006.01)

(52) Cooperative Patent Classification (CPC):
**G01K 7/02; G01K 17/10; G01T 3/00; G21C 17/108;
G21C 17/112;** Y02E 30/30

(86) International application number:
**PCT/KR2024/095070**

(87) International publication number:
**WO 2024/186182 (12.09.2024 Gazette 2024/37)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **07.03.2023 KR 20230029732**

(71) Applicant: **Korea Hydro & Nuclear Power Co., Ltd
Gyeongju-si, Gyeongsangbuk-do 38120 (KR)**

(72) Inventors:
• KIM, Kyung Gun
  **Daejeon 34071 (KR)**
• SHIN, Ho Cheol
  **Daejeon 34125 (KR)**
• CHOI, Yu Sun
  **Daejeon 34049 (KR)**
• KIM, Do Yeon
  **Daejeon 34050 (KR)**

(74) Representative: **Habermann, Hruschka &
Schnabel
Patentanwälte
Montgelasstraße 2
81679 München (DE)**

(54) **METHOD FOR CALCULATING HEAT OUTPUT BY USING IN-CORE INSTRUMENT**

(57) Disclosed is a method of calculating heat output using in-core instrumentation, the method including: measuring a core inlet temperature and a core exit temperature using the in-core instrumentation; and calculating heat output Q of a core using the following equation, the in-core instrumentation including: a first thermocouple that measures the core inlet temperature; and a second thermocouple that measures the core exit temperature.

[Equation]

$$Q = C \times m \times (T_h - T_c)$$

where, Q is a heat quantity, C is a specific heat, m is a mass flow rate, $T_h$ is a core exit temperature, and $T_c$ is a core inlet temperature.

**FIG. 1**

```
┌──────────────────────────────────┐
│  measure core inlet temperature  │ ─── S10
│    and core exit temperature     │
└──────────────────────────────────┘
              │
              ▼
┌──────────────────────────────────┐
│     calculate heat output (Q)    │ ─── S20
└──────────────────────────────────┘
```

EP 4 679 455 A1

## Description

Technical Field

The disclosure relates to a method of calculating heat output using in-core instrumentation.

Background Art

A current method of measuring the temperature of a reactor core is based on an indirect method of calculating a core inlet temperature and a core exit temperature from the measurements of resistance temperature detectors (RTD) installed in a hot leg and a cold leg of a reactor coolant system.

In an in-core instrumentation that measures the neutron flux of the reactor core, a core exit thermocouple (CET) is located at a core exit and thus directly measures the core exit temperature. However, the temperature measured by the core exit thermocouple is used in inadequate core cooling monitoring systems (ICCMS), and is thus not used directly in calculating the heat output of the core.

Disclosure

Technical Problem

An aspect of the disclosure is to provide a method of calculating heat output using an in-core instrumentation.

Technical Solution

The aspect of the disclosure is achieved by a method of calculating heat output using in-core instrumentation, the method including: measuring a core inlet temperature and a core exit temperature using the in-core instrumentation; and calculating heat output Q of a core using the following equation, the in-core instrumentation including: a first thermocouple that measures the core inlet temperature; and a second thermocouple that measures the core exit temperature.

[Equation]

$$Q = C \times m \times ( T_h - T_c )$$

where, Q is a heat quantity, C is a specific heat, m is a mass flow rate, $T_h$ is a core exit temperature, and $T_c$ is a core inlet temperature.

The core exit temperature may be calculated using at least one of an overall average method, a highest temperature method, a lowest temperature method, or a weighted average temperature.

The in-core instrumentation may include a plurality of neutron detectors, the neutron detector may include an emitter that absorbs neutrons and emits an electric current, and the emitter may be located between an end portion of the first thermocouple and an end portion of the second thermocouple.

The in-core instrumentation may further include a background detector, and the neutron detector, the background detector, the first thermocouple, and the second thermocouple may be annularly arranged.

The material of the emitter may include at least one of vanadium (V-51), cobalt (Co-59), platinum (Pt-195), and silver (Ag).

The length of the emitter may be 0.05 to 0.15 times the length of the core.

The diameter of the emitter may be 0.2 to 0.8 times the diameter of the neutron detector.

Advantageous Effects

According to the disclosure, there is provided a method of calculating heat output using in-core instrumentation.

Description of Drawings

FIG. 1 is a flowchart showing a method of calculating the heat output of a core according to an embodiment of the disclosure,

FIG. 2 illustrates in-core instrumentation using in a calculation method according to an embodiment of the disclosure,

FIG. 3 is a longitudinal-section view showing the assembly of in-core instrumentation used in a calculation method according to an embodiment of the disclosure,

FIG. 4 is a cross-section view showing the assembly of in-core instrumentation used in a calculation method according to an embodiment of the disclosure,

FIG. 5 is a view for describing the locations of a neutron detector, a core inlet thermocouple, and a core exit thermocouple used in a calculation method according to an embodiment of the disclosure,

FIG. 6 is a schematic view showing the locations of temperature detectors (RTD) placed in a hot leg and a cold leg of a reactor coolant system, and

FIG. 7 is a diagram showing the locations of temperature detectors (RTD) placed in a hot leg and a cold leg of a reactor coolant system.

Mode for Invention

Below, the disclosure will be described in more detail with reference to the accompanying drawings. The accompanying drawings are merely an example illustrated to describe the technical concept of the disclosure in more detail, and thus the technical concept of the disclosure is not limited to the accompanying drawings.

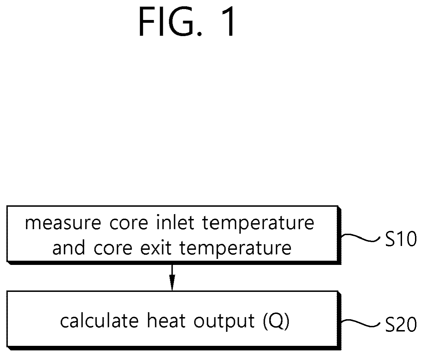

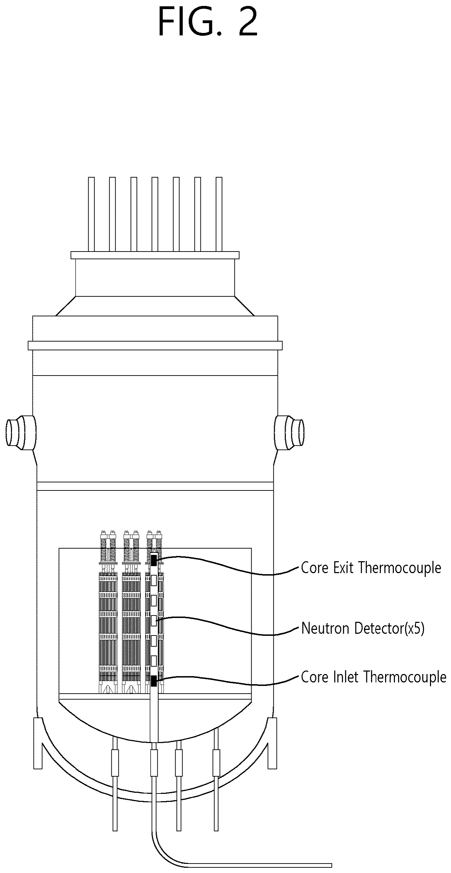

A method of calculating the heat output using in-core instrumentation will be described with reference to FIGS. 1 and 2.

FIG. 1 is a flowchart showing a method of calculating the heat output of a core according to an embodiment of the disclosure, and FIG. 2 illustrates in-core instrumentation using in a calculation method according to an embodiment of the disclosure.

In FIG. 2, a nuclear fuel assembly, a neutron detector, etc. are illustrated together.

According to this embodiment, a method of calculating the heat output using in-core instrumentation includes the steps of measuring a core inlet temperature and a core exit temperature (S10), and calculating heat output (S20).

In the step S10 of measuring the core inlet temperature and the core exit temperature, in-core instrumentation is used.

The in-core instrumentation (ICI, or an in-core instrumentation assembly) is shaped like a cylinder extending in a longitudinal direction, and includes an inner tube and an outer tube.

The in-core instrumentation may be mounted to an instrumentation guide tube on an inner side of a nuclear fuel bundle in which nuclear fuel rods are grouped in an even or odd array.

For example, the in-core instrumentation may be inserted in the guide tube on the inner side of the nuclear fuel having a fuel rod array of 14×14, 15×15, 16×16 or 17×17.

The in-core instrumentation may include thermocouples, detectors and filler wires, which are annularly arranged between the inner tube and the outer tube.

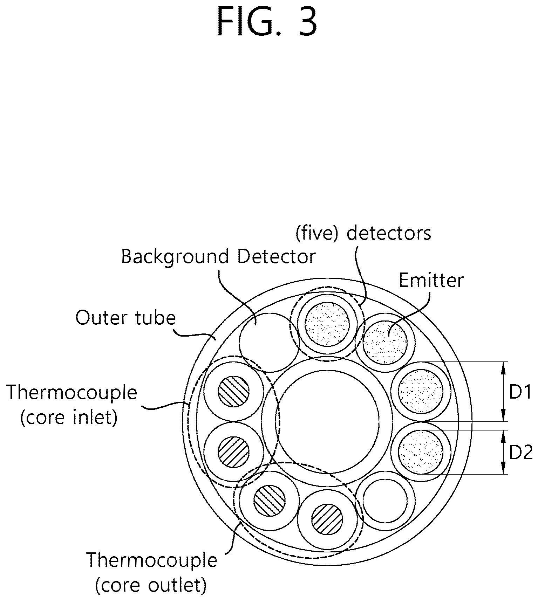

The configuration of the in-core instrumentation will be described with reference to FIGS. 3 to 5.

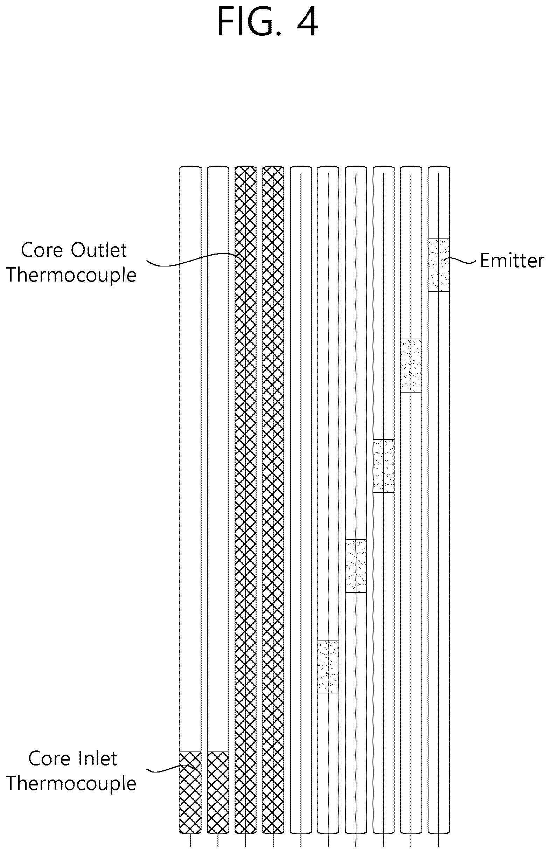

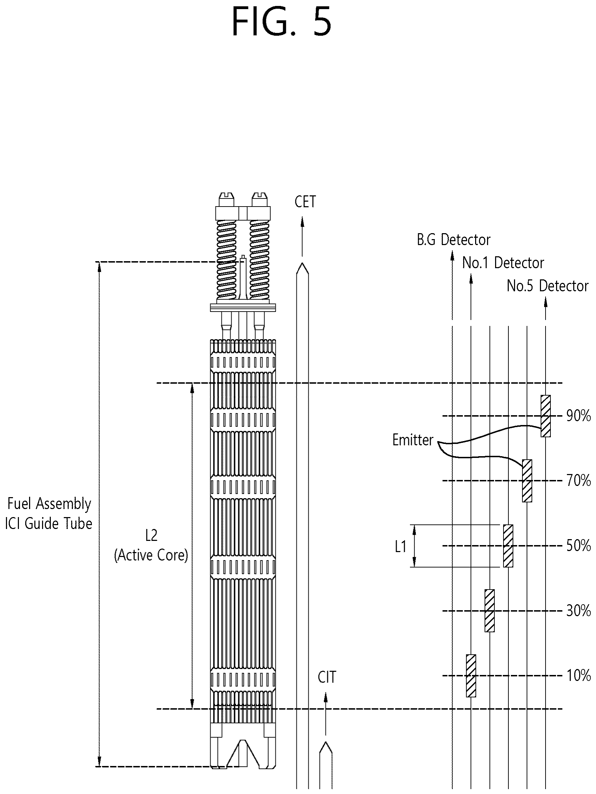

FIG. 3 is a longitudinal-section view showing the assembly of in-core instrumentation used in a calculation method according to an embodiment of the disclosure, FIG. 4 is a cross-section view showing the assembly of in-core instrumentation used in a calculation method according to an embodiment of the disclosure, and FIG. 5 is a view for describing the locations of a neutron detector, a core inlet thermocouple, and a core exit thermocouple used in a calculation method according to an embodiment of the disclosure.

A thermocouple includes two types of metals forming a pair. When an electric circuit is formed by joining one ends of the different types of metals, a thermoelectromotive force is generated between the two metals due to temperature at the junction, thereby generating a voltage which is measured and converted into the temperature.

According to an embodiment of the disclosure, a K-type thermocouple, in which chromel metal alloy and alumel metal alloy are joined, may be used.

The thermocouple includes a first thermocouple and a second thermocouple.

The first thermocouple (core inlet thermocouple, or CIT) measures the core inlet temperature, and the second thermocouple (core exit thermocouple, CET, or core outlet thermocouple) measures the core exit temperature.

The first thermocouple and the second thermocouple may directly measure the temperature at a heating contact point.

In FIGS. 4 and 5, the in-core instrumentation includes, but not limited to, a pair of first thermocouples and a pair of second thermocouples, which are located therein.

The first thermocouple and the second thermocouple may be located to be adjacent to each other or not to be adjacent to each other.

A detector includes a neutron detector and a background detector.

The neutron detector is shaped like a tube, and includes an emitter, an insulator, and a signal line, which are located inside the tube.

The tube of the neutron detector may be made of Inconel, the insulator is located between the emitter and the tube, and the signal line connected to the emitter is located at the center portion of the tube.

The neutron detector may include a plurality of neutron detectors. For convenience of description, five neutron detectors are shown in FIGS. 4 and 5, but the disclosure is not limited thereto.

The neutron detector may be a self-powered neutron detector (SPND) that does not require an external power source.

Each neutron detector includes the emitter that absorbs neutrons and emits an electric current.

The emitter may be shaped like a cylinder disposed in a longitudinal direction inside the neutron detector, but is not limited thereto.

As the material of the emitter, rhodium (Rh-103), which has been conventionally employed, may be used, and vanadium (V-51), cobalt (Co-59), platinum (Pt-195), and silver (Ag) may also be used.

As shown in FIG. 4, the diameter D2 of the emitter may be 0.4 to 0.6 times, 0.3 to 0.7 times, or 0.2 to 0.8 times the diameter D1 of the detector.

The diameter D1 of the detector may be the maximum diameter which allows the neutron detector, the background detector, the first thermocouple, and the second thermocouple to be annularly arranged within the inner diameter of the outer tube of the in-core instrumentation, and may be smaller than that maximum diameter.

As shown in FIG. 5, the length L1 of the emitter may be 0.03 to 0.12 times, 0.05 to 0.1 times, and 0.07 to 0.09 times the length of the core L2.

Here, the length L2 of the core refers to the length or height of the nuclear fuel pellets stacked.

The length L1 of the emitter may be changed to increase the signal magnitude of the electric current.

For example, the emitter in the OPR1000 type reactor may have a length of 40cm.

The emitter is located between the installation locations of the first and second thermocouples, and may be located at a height corresponding to 10%, 30%, 50%, 70% or 90% of the length of the core.

Therefore, the neutron flux may be measured according to the heights of the core.

In the step S20 of calculating the heat output, the core inlet temperature and the core exit temperature measured by the first thermocouple and the second thermocouple are applied to the following [Equation 1].

[Equation 1]

$$Q = C \times m \times ( T_h - T_c )$$

where, Q is a heat quantity, C is a specific heat, m is a mass flow rate, $T_h$ is a core exit temperature, and $T_c$ is a core inlet temperature.

The core exit temperature may be calculated by an averaging method of 45 core exit thermocouples, a highest temperature method, or a lowest temperature method, and may also be calculated by a weighted average temperature method considering a core exit flow mixing effect.

In another embodiment of the present invention, the heat output is calculated by applying the core inlet temperature and the core exit temperature to the following [Equation 2].

[Equation 2]

$$Q = \frac{( C_{out1} T_{h1} M_5 + C_{out2} T_{h2} M_6 ) - ( C_{in1} T_{c1} M_5 + C_{in2} T_{c2} M_6 )}{M_5 + M_6}$$

where, $C_{out1}$ and $C_{out2}$ are enthalpy/temperature ratios of the core exit, $C_{in1}$ and $C_{in2}$ are enthalpy/temperature ratios of the core inlet, $T_{h1}$ and $T_{h2}$ are core exit temperatures measured by a plurality of second thermocouples, $T_{c1}$ and $T_{c2}$ are core inlet temperatures measured by a plurality of first thermocouples, and $M_5$ and $M_6$ are normalized coolant mass flow rates in S/G 1 and S/G 2.

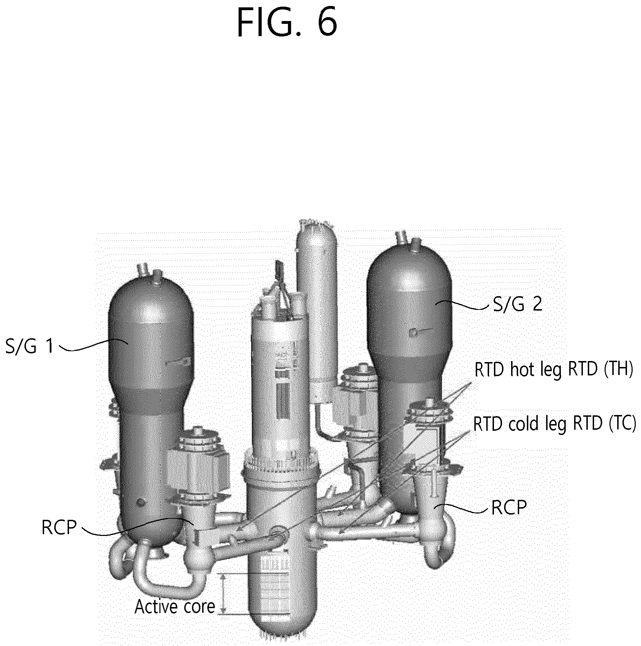

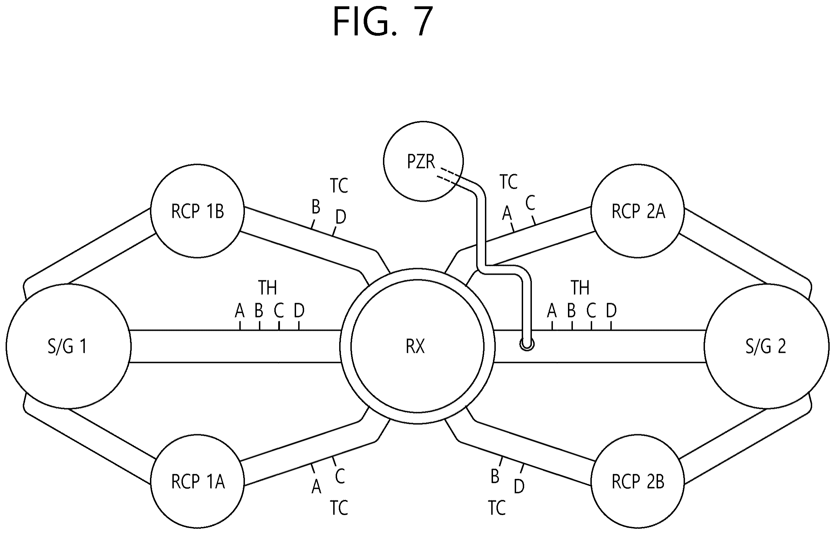

$M_5$ and $M_6$ will be described with reference to FIGS. 6 and 7.

FIG. 6 is a schematic view showing the locations of temperature detectors (RTD) placed in a hot leg and a cold leg of a reactor coolant system, and FIG. 7 is a diagram showing the locations of temperature detectors (RTD) placed in a hot leg and a cold leg of a reactor coolant system.

S/G 1 and S/G 2 refer to steam generators connected to the reactor (RX).

According to the disclosure, the heat output is calculated by directly measuring the actual temperatures at the core inlet and outlet locations through the in-core instrumentation, thereby improving accuracy.

Due to the thermal stratification at the core exit and the hot leg, the temperature measured at an RCS hot leg does not accurately reflect the core exit temperature. However, according to the disclosure, the temperature measured directly at the core exit is used to calculate the heat output.

Further, the power distribution and DNBR/LPD are calculated based on the inlet and outlet temperatures measured directly at 45 in-reactor instrument locations in the reactor compared to the temperatures measured at 2 hot legs and 4 cold legs of the conventional reactor, thereby improving a safety margin.

In addition, resistance temperature detector (RTD) type temperature measurement devices installed in the hot leg and the cold leg of the reactor coolant system are removable, thereby simplifying system equipment.

Therefore, the calculation method according to the disclosure may be used to measure the core heat output of the small module reactor (SMR) that requires equipment simplification.

In addition, the calculation method according to the disclosure may be applied immediately when signal processing equipment is added to use the core inlet temperature.

The in-core instrumentation according to the disclosure has the same outermost diameter as that of the in-core instrumentation used in the existing OPR 1000 type reactor, and is thus applicable without modifying the guide tube.

Lastly, the temperatures of the core protection calculator (CPC) and core operating limits monitoring system (COLSS) algorithms may be used as input signals by adding in-core thermocouple signal processing facilities.

Although a few embodiments of the disclosure have been described above in detail, it is apparent to a person having ordinary knowledge in the art that such embodiments are merely exemplary embodiments and do not limit the scope of the disclosure. Therefore, the substantial scope of the disclosure is defined by appended claims and their equivalents.

**Claims**

1.    A method of calculating heat output using in-core instrumentation, the method comprising:

     measuring a core inlet temperature and a core exit temperature using the in-core instrumentation; and
     calculating heat output Q of a core using the following equation,
     the in-core instrumentation comprising:

          a first thermocouple that measures the core inlet temperature; and
          a second thermocouple that measures the core exit temperature.

[Equation]

$$Q = C \times m \times (T_h - T_c)$$

     where, Q is a heat quantity, C is a specific heat, m is a mass flow rate, $T_h$ is a core exit temperature, and $T_c$ is a core inlet temperature.

2.    The method of claim 1, wherein the core exit temperature is calculated using at least one of an overall average method, a highest temperature method, a lowest temperature method, or a weighted average temperature.

3.    The method of claim 1, wherein

     the in-core instrumentation comprises a plurality of neutron detectors,
     the neutron detector comprises an emitter that absorbs neutrons and emits an electric current, and
     the emitter is located between an end portion of the first thermocouple and an end portion of the second thermocouple.

4.    The method of claim 1, wherein

     the in-core instrumentation further comprises a background detector, and
     the neutron detector, the background detector, the first thermocouple, and the second thermocouple are annularly arranged.

5. The method of claim 3, wherein a material of the emitter comprises at least one of vanadium (V-51), cobalt (Co-59), platinum (Pt-195), and silver (Ag).

6. The method of claim 3, wherein a length of the emitter is 0.05 to 0.15 times a length of the core.

7. The method of claim 3, wherein a diameter of the emitter is 0.2 to 0.8 times a diameter of the neutron detector.

# FIG. 1

```
┌─────────────────────────────────┐
│  measure core inlet temperature │ ─── S10
│   and core exit temperature     │
└─────────────────────────────────┘
                │
                ▼
┌─────────────────────────────────┐
│    calculate heat output (Q)    │ ─── S20
└─────────────────────────────────┘
```

# FIG. 2

Core Exit Thermocouple

Neutron Detector(x5)

Core Inlet Thermocouple

# FIG. 3

# FIG. 4

Core Outlet
Thermocouple

Emitter

Core Inlet
Thermocouple

# FIG. 5

# FIG. 6

# FIG. 7

EP 4 679 455 A1

## INTERNATIONAL SEARCH REPORT

| International application No. |
| --- |
| **PCT/KR2024/095070** |

| A. | CLASSIFICATION OF SUBJECT MATTER |
| --- | --- |

G21C 17/112(2006.01)i; G21C 17/108(2006.01)i; G01K 7/02(2006.01)i; G01K 17/10(2006.01)i; G01T 3/00(2006.01)i

According to International Patent Classification (IPC) or to both national classification and IPC

| B. | FIELDS SEARCHED |
| --- | --- |

Minimum documentation searched (classification system followed by classification symbols)

G21C 17/112(2006.01); G01K 1/14(2006.01); G01K 7/02(2006.01); G21C 17/00(2006.01); G21C 17/02(2006.01); G21C 17/08(2006.01); G21C 17/10(2006.01); G21C 17/108(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 열출력(heat output), 노내계측기(in-core instrument), 열전대(thermocouple), 온도 (temperature), 검출기(detector)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | KR 10-1621236 B1 (WOOJIN INC. et al.) 17 May 2016 (2016-05-17)<br>See paragraphs [0001] and [0024]; and figures 4-5. | 1-7 |
| A | JP 2005-172474 A (GLOBAL NUCLEAR FUEL-JAPAN CO., LTD.) 30 June 2005 (2005-06-30)<br>See paragraphs [0001]-[0010] and [0024]-[0025]; and figures 1 and 6. | 1-7 |
| A | KR 10-2013-0123940 A (KOREA HYDRO & NUCLEAR POWER CO., LTD.) 13 November 2013 (2013-11-13)<br>See paragraphs [0001] and [0026]; and figures 3-4. | 1-7 |
| A | JP 2001-074875 A (SHIKOKU ELECTRIC POWER CO., INC. et al.) 23 March 2001 (2001-03-23)<br>See entire document. | 1-7 |
| A | US 8444317 B2 (LEE, Yeu Yong) 21 May 2013 (2013-05-21)<br>See entire document. | 1-7 |

☐ Further documents are listed in the continuation of Box C.    ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **13 June 2024** | **13 June 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**EP 4 679 455 A1**

## INTERNATIONAL SEARCH REPORT
### Information on patent family members

| Patent document cited in search report | | | Publication date (day/month/year) | Patent family member(s) | | | Publication date (day/month/year) |
|---|---|---|---|---|---|---|---|
| KR | 10-1621236 | B1 | 17 May 2016 | CN | 105387948 | A | 09 March 2016 |
| | | | | FR | 3025048 | A1 | 26 February 2016 |
| | | | | FR | 3025048 | B1 | 18 June 2021 |
| | | | | FR | 3029344 | A1 | 03 June 2016 |
| | | | | FR | 3029344 | B1 | 02 July 2021 |
| | | | | JP | 2016-045191 | A | 04 April 2016 |
| | | | | KR | 10-1671312 | B1 | 01 November 2016 |
| | | | | KR | 10-2016-0025088 | A | 08 March 2016 |
| | | | | KR | 10-2016-0025640 | A | 09 March 2016 |
| | | | | US | 2016-0055926 | A1 | 25 February 2016 |
| JP | 2005-172474 | A | 30 June 2005 | None | | | |
| KR | 10-2013-0123940 | A | 13 November 2013 | KR | 10-1484000 | B1 | 19 January 2015 |
| JP | 2001-074875 | A | 23 March 2001 | None | | | |
| US | 8444317 | B2 | 21 May 2013 | KR | 10-1034386 | B1 | 16 May 2011 |
| | | | | US | 2012-0076170 | A1 | 29 March 2012 |

Form PCT/ISA/210 (patent family annex) (July 2022)